(19) Europäisches Patentamt
European Patent Office
Office européen des brevets

(11) Veröffentlichungsnummer: **0 009 699**
**B1**

(12) # EUROPÄISCHE PATENTSCHRIFT

(45) Veröffentlichungstag der Patentschrift: **11.11.81**

(51) Int. Cl.³: **C 07 C 69/88**, C 07 C 67/00, C 07 C101/52

(21) Anmeldenummer: **79103467.1**

(22) Anmeldetag: **17.09.79**

(54) **Verfahren zur Herstellung weitgehend wasserfreier und Hydroxybenzoesäure-freier Alkalisalze von Hydroxybenzoesäureestern.**

(30) Priorität: **27.04.79 DE 2917273**
**30.09.78 DE 2842807**

(43) Veröffentlichungstag der Anmeldung: **16.04.80 (Patentblatt 80/08)**

(45) Bekanntmachung des Hinweises auf die Patenterteilung: **11.11.81 Patentblatt 81/45**

(84) Benannte Vertragsstaaten: **CH DE FR GB NL SE**

(56) Entgegenhaltungen:
**DE - B - 1 907 230**
**DE - C - 713 690**

**ARCHIV DER PHARMAZIE UND BERICHTE DER DEUTSCHEN PHARMAZEUTISCHEN GESELLSCHAFT**
**Band 267 (1929) Seite 684**

(73) Patentinhaber: **BAYER AG**
**Zentralbereich Patente, Marken und Lizenzen**
**D-5090 Leverkusen 1, Bayerwerk (DE)**

(72) Erfinder: **Schnegg, Peter, Dr.**
**Heidberger Strasse 44**
**D-5068 Odenthal (DE)**
Erfinder: **Rapp, Walter, Dr.**
**Walter-Flex-Strasse 13**
**D-5090 Leverkusen 1 (DE)**
Erfinder: **Vosteen, Bernhard, Dr.**
**Roggendorfstrasse 49**
**D-5000 Köln 80 (DE)**

**EP 0 009 699 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

Verfahren zur Herstellung weitgehend wasserfreier und Hydroxybenzoesäure-freier Alkalisalze von Hydroxybenzoesäureestern

Die Erfindung betrifft ein Verfahren zur Herstellung weitgehend wasserfreier und Hydroxybenzoesäure-freier Alkalisalze von Hydroxybenzoesäureestern.

Es ist bekannt, die Alkalisalze von Hydroxybenzoesäureestern durch Zusammengeben einer ätherischen Lösung des Esters und einer konzentrierten methanolischen Lösung eines Alkalihydroxids herzustellen (Archiv der Pharmazie 267, 684 (1929)). Dieses offensichtlich unwirtschaftliche und wegen der Brennbarkeit der Lösungsmittel gefährliche Verfahren liefert die Alkalisalze der niederen Alkylester der 4-Hydroxybenzoesäure nicht völlig wasserfrei und ist auf die höheren Ester überhaupt nicht anwendbar (DRP 713 690).

Ferner ist bekannt, wasserfreie Alkalisalze von Hydroxybenzoesäureestern durch Umsetzung eines Hydroxybenzoesäureesters mit einem Alkaliphenolat in einem inerten organischen Lösungsmittel herzustellen (DE-OS 2 044 705). Dieses Verfahren setzt die Herstellung eines wasserfreien Alkaliphenolates voraus und führt zu Produkten, die wegen der möglichen Kontamination mit physiologisch bedenklichen organischen Lösungsmitteln, wie beispielsweise aromatischen Kohlenwasserstoffen, und mit dem Schwerflüchtigen, ätzenden Phenol als Zusatzstoff zu Lebensmitteln (US 2 046 324) ungeeignet erscheinen.

Es ist weiter bekannt, Alkalisalze von Hydroxybenzoesäureestern durch inniges Vermischen des Esters mit der äquivalenten Menge an festem Alkalihydroxid unter Zugabe von nur wenig Wasser herzustellen. Dabei wird solange ein dickflüssiger Zustand durchlaufen, bis infolge der Neutralisationswärme das Wasser verdampft ist (DRP 713 690). Hierbei besteht jedoch die Gefahr der Verseifung der Estergruppe des Hydroxybenzoesäureesters durch das Wasser im dickflüssigen Zustand unter dem Einfluß der Neutralisationswärme (DE-AS 1 907 230).

In einem anderen Verfahren zur Gewinnung von Alkalisalzen eines Hydroxybenzoesäureesters wird die Neutralisation in Wasser bei tiefer Temperatur durchgeführt und ein Hydrat des Alkalisalzes des Hydroxybenzoesäureesters abgeschieden, getrocknet und bei nur langsam steigender Temperatur derart entwässert, daß das Hydrat nicht schmilzt (DE-AS 1 907 230). Bei diesem Verfahren werden Ausbeuten von etwa 60 % der theoretischen Ausbeute erhalten. Es sind lange Trockenzeiten von 11 bis 18 Stunden notwendig.

Es wurde ein Verfahren zur Herstellung von weitgehend wasserfreien und Hydroxybenzoesäure-freien Alkalisalzen von Hydroxybenzoesäureestern der Formel (I)

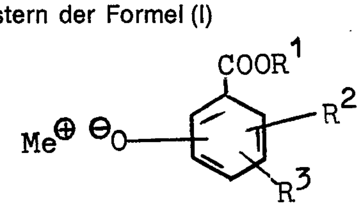

(I)

in der

$R^1$ Alkyl, Alkenyl, Cycloalkyl oder Aralkyl bedeutet,

$R^2$ und $R^3$ gleich oder verschieden sind und für Wasserstoff, Halogen, Hydroxy, Amino, Alkylamino, Alkyl, Alkoxy, Aralkyl oder Aryl stehen und

Me ein Alkalimetall bedeutet, gefunden, das dadurch gekennzeichnet ist, daß man eine Lösung oder Suspension eines Hydroxybenzoesäureesters der Formel (II)

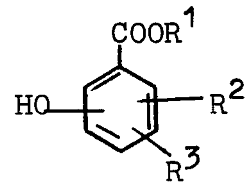

(II)

in der

$R^1$, $R^2$ und $R^3$ die oben genannte Bedeutung haben, mit einem Alkalihydroxid bei −10 bis + 50 °C bis zu einem Neutralisationsgrad von 0,95 bis 1,05 neutralisiert, wobei der Neutralisationsgrad 1,00 den Äquivalenzpunkt der Neutralisation der phenolischen OH-Gruppe durch das Alkalihydroxid bedeutet, und die dabei erhaltene Lösung oder Suspension des Alkalisalzes des Hydroxybenzoesäureesters so rechtzeitig einer an sich bekannten produktschonenden Trocknung zuführt, daß der Gehalt an Hydroxybenzoesäure, bzw. deren Alkalisalz in dieser Lösung oder Suspension den Wert von 1 Gew.-%, bezogen auf die in dieser Lösung oder Suspension enthaltene Menge an Alkalisalz des Hydroxybenzoesäureesters, nicht erreicht.

Als Alkylreste seien beispielsweise solche mit 1 bis 20 Kohlenstoffatomen genannt, wie Methyl, Äthyl, Propyl, Isopropyl, Butyl, Isobutyl, Hexyl, Heptyl, Octyl, Isooctyl, Decyl, Dodecyl, Hexadecyl oder Eicosyl. Bevorzugt sind Alkylreste mit 1 bis 8 Kohlenstoffatomen, ganz besonders bevorzugt solche mit 1 bis 4 Kohlenstoffatomen.

Als Alkenylreste seien beispielsweise solche mit 3 bis 8 Kohlenstoffatomen genannt, wie Allyl, Butenyl, Hexenyl oder Octenyl. Bevorzugt sind Alkenylreste mit 3 bis 4 Kohlenstoffatomen, besonders bevorzugt ist der Allylrest.

Als Cycloalkylreste seien beispielsweise solche mit 4 bis 8 Kohlenstoffatomen genannt, wie Cyclobutyl, Cyclopentyl, Cyclohexyl, Cycloheptyl oder Cyclooctyl. Bevorzugte Cycloalkylreste sind solche mit 5 bis 6 Kohlenstoffatomen.

Als Aralkylreste seien beispielsweise solche mit 1 bis 2 Kohlenstoffatomen im aliphatischen Teil und 6 bis 12 Kohlenstoffatomen im aromatischen Teil genannt, wie Benzyl, Phenyläthyl oder Biphenylmethyl. Bevorzugtes Aralkyl ist Benzyl.

Als Halogen sei beispielsweise Fluor, Chlor, Brom oder Jod, bevorzugt Chlor oder Brom, genannt.

Als Alkylaminosubstituenten seien beispielsweise solche mit 1 bis 4 Kohlenstoffatomen genannt, wie Methylamino, Dimethylamino, Äthylamino, Diäthylamino, Propylamino oder Butylamino.

Als Alkoxysubstituenten seien beispielsweise solche mit 1 bis 4 Kohlenstoffatomen genannt, wie Methoxy, Äthoxy, Propyloxy oder Butyloxy.

Als Arylsubstituenten seien beispielsweise solche mit 6 bis 12 Kohlenstoffatomen genannt, wie Phenyl, Biphenyl oder Naphthyl. Bevorzugtes Aryl ist Phenyl.

Aromatische Substituenten können ihrerseits durch Hydroxy, Amino oder Halogen, beispielsweise Fluor, Chlor, Brom oder Jod, substituiert sein.

Als Alkalimetall sei beispielsweise Natrium, Kalium, Lithium, bevorzugt Natrium oder Kalium und besonders bevorzugt Natrium, genannt.

Als weitgehend wasserfreie Alkalisalze von Hydroxybenzoesäureestern seien beispielsweise solche genannt, die nicht mehr als 5 Gew.-%, bevorzugt nicht mehr als 2 Gew.-%, besonders bevorzugt nicht mehr als 0,5 Gew.-% Wasser enthalten.

Als weitgehend Hydroxybenzoesäure-freie Alkalisalze von Hydroxybenzoesäureestern seien beispielsweise solche genannt, die einen Gehalt von nicht mehr als 3 Gew.-% an Hydroxybenzoesäure bzw. deren Alkalisalz haben. Bevorzugte Stoffe der Formel (I) sind solche, die einen Gehalt von nicht mehr als 1 Gew.-%, besonders bevorzugt solche, die einen Gehalt von nicht mehr als 0,35 Gew.-% an Hydroxybenzoesäure bzw. deren Alkalisalz haben.

Bevorzugte Hydroxybenzoesäureester zur Herstellung einer Lösung oder Suspension ihrer Alkalisalze sind solche der Formel (II)

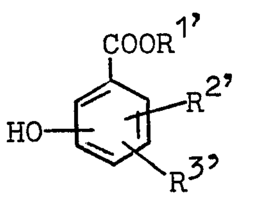

(II)

bei denen

$R^{1'}$ Alkyl, Allyl, Cyclohexyl oder Benzyl bedeutet und $R^{2'}$ und $R^{3'}$ gleich oder verschieden sind und für Wasserstoff, Chlor, Brom, Hydroxy, Methyl oder Methoxy stehen.

Ganz besonders bevorzugte Hydroxybenzoesäureester zur Herstellung einer Lösung oder Suspension ihrer Alkalisalze sind solche der Formel (II), in der $R^1$ Alkyl, Allyl, Cyclohexyl oder Benzyl bedeutet und $R^2$ und $R^3$ für Wasserstoff stehen.

Als erfindungsgemäß einsetzbare Hydroxybenzoesäureester seien beispielsweise genannt: Die Methyl-, Äthyl-, Propyl-, Isopropyl-, Butyl-, Isobutyl-, Allyl-, Cyclopentyl-, Cyclohexyl- oder Benzyl-Ester der 2-Hydroxy-, 3-Hydroxy-, 4-Hydroxy-, 3-Methyl-2-hydroxy-, 2-Hydroxy-3-methyl-5-chlor-, 2-Hydroxy-5-tert.-butyl-, 2,4-Dihydroxy-, 2-Hydroxy-3-methoxy-, 3-Amino-4-hydroxy-, 4-Amino-2-hydroxy-, 5-Chlor-2-hydroxy-, 3,5-Dihydroxy- oder 3-Chlor-4-hydroxybenzoesäure.

Hydroxybenzoesäureester sind bekannt und können beispielsweise durch Veresterung von Hyproxybenzoesäuren mit geeigneten Alkoholen hergestellt werden (J. Org. Chem. 2, 253 (1937); Houben-Weyl, Methoden der organischen Chemie, Band VIII, Seite 468, 525, 544, Georg-Thieme-Verlag, Stuttgart (1952)).

Als Alkalihydroxide zur Neutralisation der Hydroxybenzoesäureester seien beispielsweise Lithiumhydroxid, Natriumhydroxid oder Kaliumhydroxid, bevorzugt Natriumhydroxid oder Kaliumhydroxid, ganz bevorzugt Natriumhydroxid genannt.

Als Lösungs- oder Suspendiermittel seien solche genannt, in denen die gewünschte Salzbildung an der phenolischen OH-Gruppe stattfindet, ohne daß sie selbst unter den Reaktionsbedingungen der Salzbildung und der späteren Trocknung verändert werden, wie niedere aliphatische Alkohole, Wasser oder Mischungen von niederen aliphatischen Alkoholen mit Wasser. Als niedere aliphatische Alkohole seien besonders solche mit 1-4 Kohlenstoffatomen, wie Methanol, Äthanol, Propanol, Isopropanol, Butanol, Iso-butanol oder tert. Butanol genannt. Besonders bevorzugt als Lösungs- oder Suspendiermittel ist aus ökonomischen, ökologischen und sicherheitstechnischen Erwägungen das Wasser.

Die Neutralisation wird im Temperaturbereich von −10 °C bis + 50 °C, vorzugsweise im Bereich von + 15 bis + 25 °C, durchgeführt.

Die Konzentration des Alkalisalzes eines Hydroxybenzoesäureesters in der Lösung oder Suspension, in der die Neutralisation vorgenommen wird, und die der Trocknung zugeführt wird, kann 10-80 Gew.-%, vorzugsweise 25-60 Gew.-%, betragen.

In einer bevorzugten Ausführungsform des erfindungsgemäßen Verfahrens wird die Konzentration des Hydroxybenzoesäureesters so eingestellt, daß im Verlaufe der Neutralisierung eine Lösung entsteht. Hierbei kann die Konzentration des Alkalisalzes eines Hydroxybenzoesäureesters in der Lösung beispielsweise 0,5 bis 10, bevorzugt 1 bis 5 Gew.-% unter der jeweiligen Sättigungskonzentration liegen.

Eine Lösung oder Suspension eines Hydroxybenzoesäureesters wird im erfindungsgemäßen Verfahren bis zu einem Neutralisationsgrad von 0,95 bis 1,05 neutralisiert, wobei der Neutralisationsgrad 1,00 den Punkt bedeutet, an dem die Menge phenolischer OH-Gruppe und die Menge Alkalihydroxid einander genau äquivalent sind.

Bevorzugt ist ein Neutralisationsgrad von 0,97 bis 1,03 und besonders bevorzugt ein Neutralisationsgrad von 0,98 bis 1,00.

Der Vorgang der Neutralisation des gelösten oder suspendierten Hydroxybenzoesäureesters kann in einer oder in zwei Stufen vorgenommen werden. Hierbei kann diskontinuierlich oder kontinuierlich gearbeitet werden. Bei zweistufiger Neutralisation können beide Stufen unabhängig voneinander diskontinuierlich oder kontinuierlich durchgeführt werden.

Bei einstufiger Neutralisation und wenn zu Beginn der Neutralisation eine Suspension des Hydroxybenzoesäureesters vorliegt, muß die Zugabe von Alkalihydroxid im allgemeinen unter ständiger pH-Kontrolle vorgenommen werden. Dadurch wird gewährleistet, daß zu keiner Zeit überschüssiges Alkalihydroxid, bezogen auf den gelösten Hydroxybenzoesäureester, vorhanden ist. Wird das Alkalihydroxid rascher zugegeben, als sich der suspendierte Hydroxybenzoesäureester löst, stellt sich ein so hoher pH-Wert ein, daß das in Lösung befindliche Alkalisalz des Hydroxybenzoesäureesters bereits während der Neutralisation eine merkliche Verseifund zur Hydroxybenzoesäure bzw. deren Salz erleidet.

Bei der zweistufigen Neutralisation kann nun infolge der relativ hohen Lösegeschwindigkeit bei niederen Neutralisationsgraden der größte Teil des zur vollständigen Neutralisation benötigten Alkalihydroxids ohne pH-Kontrolle zur Suspension des vorgelegten Hydroxybenzoesäureesters gegeben werden, wenn diese Zugabe in einem nich zu kleinen Zeitraum gleichmäßig durchgeführt wird. Im allgemeinen kommt als Zeitraum ein Bereich von etwa 10 bis 40 Minuten in Frage.

Die Durchführung der Neutralisation in zwei Stufen ist eine bevorzugte Variante des erfindungsgemäßen Verfahrens, wobei in der ersten Stufe ein Neutralisationsgrad von 0,90 bis 0,97, bevorzugt 0,94 bis 0,96, erreicht wird und in der zweiten Stufe die Neutralisation vervollständigt wird.

Hydroxybenzoesäureester sind bei den bevorzugten hohen Konzentrationen nahe der Sättigungskonzentration in der wäßrigen Lösung ihrer Alkalisalze sehr viel stärker löslich als in Wasser. So kann beispielsweise bei einem Neutralisationsgrad von 0,90 bis 0,97 und einer geeigneten, stoffabhängigen Konzentration eine vollständige Auflösung des anfangs suspendierten Hydroxybenzoesäureesters erreicht werden, wobei die Lösung noch nicht neutralisierten Hydroxybenzoesäureester enthält. Beispielsweise ist der 4-Hydroxybenzoesäure-n-propylester in einer Lösung seines Natriumsalzes mehr als 100-fach stärker löslich als in Wasser.

Die Lösung mit einem Neutralisationsgrad von 0,90 bis 0,97 kann, gegebenenfalls nach einer im technischen Verfahren notwendig werdenden Zwischenlagerung, unmittelbar vor der beabsichtigten Trocknung vollständig neutralisiert werden.

Diese zweite Neutralisationsstufe sollte durch eine pH-Kontrolle überwacht werden, sie kann jedoch beliebig rasch durchgeführt werden, da sie in homogener Phase als Ionenreaktion verläuft.

Nach der Neutralisation von Hydroxybenzoesäureestern in zwei Stufen wird unmittelbar nach Vervollständigung der Neutralisation ein deutlich geringerer Gehalt an Hydroxybenzoesäure als bei Neutralisation in nur einer Stufe beobachtet.

Die erfindungsgemäß hergestellte Lösung oder Suspension eines Alkalisalzes eines Hydroxybenzoesäureesters wird zur Gewinnung der weitgehend wasserfreien und Hydroxybenzoesäure-freien Alkalisalze der Hydroxybenzoesäureester einer produktschonenden Trocknung unterworfen. Produktschonende konvektive Trocknungsverfahren oder Kontakttrocknungsverfahren zeichnen sich beispielsweise durch kurze Trocknungszeiten, niedrige Trocknungstemperaturen, Entfernung der Lösungs- oder Suspendiermittels im Vakuum oder durch eine Kombination solcher Merkmale aus und sind dem Fachmann bekannt.

Die Trocknung kann beispielsweise in einem Zerstäubungstrockner üblicher Bauart mit offenem oder geschlossenem Gassystem in sauerstofffreier oder sauerstoffhaltiger Atmosphäre im üblichen Temperaturbereich von etwa 130 bis 250 °C bei üblichem Temperaturgefälle zwischen Gaseintritt und Gasaustritt von etwa 50 bis 200 °C erfolgen.

Die Trocknung kann jedoch auch durch Dünnschichtkontakttrocknung auf einem Walzentrockner bei Normaldruck oder vermindertem Druck, vorzugsweise bei vermindertem Druck, bei üblichen Heizmitteltemperaturen von beispielsweise 80 bis 180 °C erfolgen. Im Fall der Vakuum-Walzentrocknung können übliche Drucke, vorzugsweise unter 100 mbar, angewendet werden.

Die Trocknung kann jedoch auch durch Dickschichtkontakttrocknung unter Vakuum in einem produktumwälzenden Trockner erfolgen. Auch hier können übliche Heizmitteltemperaturen, beispielsweise 80 bis 180 °C, und übliche Drucke, vorzugsweise unter 100 mbar, angewendet werden.

Das Hydroxybenzoesäureester-Alkalisalz verläßt den Trockner als weitgehend wasserfreier Stoff, d.h. im erfindungsgemäßen Sinn mit einem Wassergehalt von nicht mehr als 5 Gew.-%, bevorzugt nicht mehr als 2 Gew.-%, besonders bevorzugt nicht mehr als 0,5 Gew.-% im getrockneten Hydroxybenzoesäureester-Alkalisalz.

Die Lösung oder Suspension eines Alkalisalzes eines Hydroxybenzoesäureesters mit einem Neutralisationsgrad von kleiner als 1,00 enthält eine kleine Menge Hydroxybenzoesäureester mit freier phenolischer OH Gruppe. Solche Hydroxybenzoesäureester haben niedrige Schmelzpunkte, die zum Teil unter 100 °C liegen (Ullmann's Enzyklopädie der Technischen Chemie, Band 13, Seiten 90 und 93, Urban & Schwarzenberg, München/Berlin, 1962). Es mußte erwartet werden, daß bereits ein geringer Anteil von 1-2 % an Hydroxybenzoesäureester mit freier phenolischer OH-Gruppe bei den üblichen hohen Trockentemperaturen zum Verkleben der getrockneten Teilchen und damit zu Verkrustungen in den Trockenapparaten, führt. Während diese Erwartung bei einem Neutralisationsgrad von kleiner als 0,95 auch in immer stärkerem Maße zutrifft und von der Art des Esters, insbesondere von dessen Schmelzpunkt abhängig ist, ist es überraschenderweise nach dem erfindungsgemäßen Verfahren möglich, Lösungen oder Suspensionen eines Alkalisalzes eines Hydroxybenzoesäureesters mit einem Neutralisationsgrad von 0,95 bis 1,00, bevorzugt 0,97-1,00, besonders bevorzugt 0,98-1,00 in ein nichtklebendes getrocknetes Gut zu überführen.

Die Lösung oder Suspension eine Alkalisalzes eines Hydroxybenzoesäureesters wird erfindungsgemäß der produktschonenden Trocknung so rechtzeitig zugeführt, daß der Gehalt an Hydroxybenzoesäure, bzw. deren Alkalisalz in dieser Lösung oder Suspension den Wert von 1 Gew.-%, bezogen auf die in dieser Lösung oder Suspension enthaltene Menge an Alkalisalz des Hydroxybenzoesäureesters, nicht erreicht. Bevorzugt wird die Lösung oder Suspension eines Alkalisalzes eines Hydroxybenzoesäureesters der produktschonenden Trocknung so rechtzeitig zugeführt, daß der Gehalt an Hydroxybenzoesäure, bzw. deren Alkalisalz in dieser Lösung oder Suspension einen Wert von 0,01 bis 0,95 Gew.-%, besonders bevorzugt 0,03 bis 0,5 Gew.-%, bezogen auf die in dieser Lösung oder Suspension enthaltene Menge an Alkalisalz des Hydroxybenzoesäureesters, enthält. Selbstverständlich wird im Sinne des Erfindungsgedankens ein möglichst geringer Gehalt an Hydroxybenzoesäure bzw. deren Alkalisalz in dieser Lösung oder Suspension angestrebt.

Die rechtzeitige Zuführung der neutralisierten Lösung oder Suspension eines Alkalisalzes eines Hydroxybenzoesäureesters im Sinne der Erfindungsgedankens ist mit den übrigen Merkmalen des erfindungsgemäßen Verfahrens, beispielsweise mit der Temperatur, dem Neutralisationsgrad oder der Konzentration, verknüpft. Beispielsweise wird in einer 50 %igen wäßrigen Lösung des Natriumsalzes des 4-Hydroxy-benzoesäure-n-propylesters mit einem Neutralisationsgrad von 0,95 bei 20 °C der Grenzwert, der einen Gehalt von 1 Gew.-% 4-Hydroxybenzoesäure, bzw. deren Natriumsalz, im getrocknetem Natriumsalz des 4-Hydroxybenzoesäure-n-propylesters verursacht, erst nach 15 Tagen erreich. Wird die Neutralisation bei höherer Temperatur, beispielsweise bis zu 50 °C, und bis zu einem höheren Neutralisationsgrad, beispielsweise bis zu einem Neutralisationsgrad von 1,05, durchgeführt, so bedeutet die rechtzeitige Zuführung der neutralisierten Lösung oder Suspension zur Trocknung einen Zeitraum von beispielsweise 1 bis 12 Stunden. Es liegt selbstverständlich ebenfalls innerhalb der Erfindungsgedankens, daß eine Lösung oder Suspension eines Alkalisalzes eines Hydroxybenzoesäureesters, die bei niedriger Temperatur, beispielsweise 20 °C, bis zu einem Neutralisationsgrad von beispielsweise 0,95 neutralisiert wurde, durch andere als die beschriebenen Trocknungsverfahren getrocknet werden kann, wobei diese anderen Trocknungsverfahren gegebenenfalls auch durch längere Trocknungszeiten und/oder erhöhte Trocknungstemperaturen gekennzeichnet sein können.

Es ist überraschend, daß die Alkalisalze der Hydroxybenzoesäureester im erfindungsgemäßen Verfahren aus einer Lösung oder Suspension getrocknet werden können und dabei weitgehend Hydroxybenzoesäure-frei erhalten werden, obwohl bekannt ist, daß in Lösungen eines Alkalisalzes eines Hydroxybenzoesäureesters die Estergruppe bei erhöhter Temperatur rasch verseift wird (DE-AS 1 907 230).

Es ist weiterhin überraschend, daß Lösungen eines Alkalisalzes eines Hydroxybenzoesäureesters mit einem Neutralisationsgrad bis zu 1,00, vorzugsweise von 0,98 bis 1,00, bei den genannten Bedingungen getrocknet werden können, ohne daß der Anteil an nicht durch Salzbildung an der phenolischen OH-Gruppe gebundenem Hydroxybenzoesäureester zum Verkleben der getrockneten Teilchen und damit zu Verkrustungen in den Trockenapparaten führt.

Die Alkalisalze von Hydroxybenzoesäureestern finden Verwendung bei der Herstellung von Rohmaterialien für synthetische Fasern (DE-OS 2 044 705), bei der Herstellung von Erdalkalimetallsalzen und anderen Metallsalzen der Hydroxybenzoesäureester (US 2 046 324 ; schwedisches Patent 120 451), sowie aufgrund ihrer mikrobiziden Wirkung als Zusatzstoffe zu Lebensmitteln, Pharmazeutika, Kosmetika und sonstigen durch Mikrobenbefall gefährdeten tierischen und pflanzlichen Produkten (US 2 046 324).

Für die Verwendung der Hydroxybenzoesäureester als Zusatzstoffe in Lebensmitteln gibt es in der ZusatzstoffVerkehrsverordnung vom 20.12.1977 (Bundesgesetzblatt I 1977, Seite 2653 ff.) Angaben zum Gehalt an nicht veresterter Hydroxybenzoesäure. Beispielsweise schreibt die genannte Verordnung maximale Gehalte von 0,35 Gew.-% p-Hydroxybenzoesäure im zugehörigen Ethyl- beziehungsweise n-Propyl-Ester und 0,7 Gew.-% p-Hydroxybenzoesäure im zugehörigen Methylester vor.

Beispiel 1

873,3 g 4-Hydroxybenzoesäuremethylester wurden in 2 000 ml Wasser suspendiert und bei 20 °C mit 301 ml 50 %iger Natronlauge neutralisiert, wobei gegen Ende der Natronlaugezugabe so langsam zudosiert wurde, daß der sich nach vollständiger Neutralisation einstellende pH-Wert von 12,1 zu keiner Zeit überschritten wurde. Die so nach etwa 90 Minuten erhaltene Lösung wurde zur Hälfte sofort im Zerstäubungstrockner bei einer Lufteintrittstemperatur von 220 °C und einer Luftaustrittstemperatur von 130 °C getrocknet (a). Die zweite Hälfte wurde dem Zerstäubungstrockner nach einer Lagerung von 92 Stunden und einer Lagertemperatur von 20 °C zugeführt (b). Man erhält das Natriumsalz des 4-Hydroxybenzoesäuremethylesters im Fall a) mit 0,4 Gew.-% 4-Hydroxybenzoesäure und 1,3 Gew.-% Wasser, im Fall b) mit 2,5 Gew.-% 4-Hydroxybenzoesäure und 4,2 Gew.-% Wasser.

Beispiel 2

456,6 g 4-Hydroxybenzoesäuremethylester

wurden in 1 050 ml Wasser suspendiert und bei 20 °C mit 157 ml 50 %iger Natronlauge in zwei Stufen neutralisiert. In der ersten Stufe wurden 95 % der Natronlauge in 30 Minuten zugesetzt. Nach 97 stündiger Lagerung bei 20 °C wurde die Lösung nach Zugabe der restlichen 5 % Natronlauge im Zerstäubungstrockner getrocknet. Das den Trockner verlassende etwa 80 bis 120 °C heiße Salz ist hygroskopisch und wird unter Ausschluß von Luftfeuchtigkeit gekühlt und verpackt. Der Gehalt an 4-Hydroxybenzoesäure im Natriumsalz des 4-Hydroxybenzoesäuremethylesters beträgt 0,5 Gew.-%, der Wassergehalt beträgt 1,9 Gew.-%.

Beispiel 3

304,4 g 4-Hydroxybenzoesäuremethylester wurden suspendiert in 700 ml Wasser und mit 100 ml 50 %iger Natronlauge in 20 Minuten versetzt. Sobald nach weiteren 20 Minuten vollständige Lösung eingetreten war, wurden weitere 4 ml 50 %iger Natronlauge zugetropft, wodurch ein Neutralisationsgrad von 0,99 bei einem pH-Wert von 11,5 erreicht wurde. Nach der Zerstäubungstrocknung in einem Laborzerstäubungstrockner betrug der 4-Hydroxybenzoesäuregehalt im Natriumsalz des 4-Hydroxybenzoesäuremethylesters 0,2 Gewichtsprozent; der Wassergehalt betrug 0,2 Gew.-%.

Beispiel 4

891 g 4-Hydroxybenzoesäure-n-propylester wurden in 712 ml Wasser mit 260 ml 50 %iger Natronlauge in zwei Stufen neutralisiert. Nach der ersten Stufe der Neutralisation (247 ml Natronlauge) wurde die erhaltene klare Lösung 96 Stunden lang bei 20 °C gelagert. Nach Vervollständigung der Neutralisation (13 ml Natronlauge) ergab die Zerstäubungstrocknung ein Produkt mit einem Gehalt an 4-Hydroxybenzoesäure von 0,1 Gewichtsprozent und einem Wassergehalt von 0,3 Gew.-%.

Beispiel 5

498 g 4-Hydroxybenzoesäureäthylester wurden in 874 ml Wasser suspendiert und bei 18-20 °C innerhalb von 30 Minuten mit 150 ml 50 %iger Natronlauge bis zu einem Neutralisationsgrad von 0,95 versetzt. Nach weiteren 20 Minuten, als eine klare Lösung vorlag, wurden weitere 4 % der Natronlauge (6 ml) unter Rühren hinzugefügt.

Die unmittelbar anschließende Zerstäubungstrocknung lieferte das Natriumsalz des 4-Hydroxybenzoesäureäthylesters mit einem Gehalt von 0,05 Gewichtsprozent an 4-Hydroxybenzoesäure und einem Wassergehalt von 1,5 Gewichtsprozent.

Beispiel 6

304,4 g 4-Hydroxybenzoesäuremethylester wurden in 740 ml Wasser suspendiert und bei 20 °C durch Zugabe von 142 ml 50 %iger Kalilauge innerhalb 20 Minuten in Lösung gebracht. Mit weiteren 6 ml der 50 %igen Kalilauge wurde vollständig neutralisiert (pH 12,1). Die Zerstäubungstrocknung lieferte das rein weiße Kaliumsalz des 4-Hydroxybenzoesäuremethylesters mit einem Gehalt an 4-Hydroxybenzoesäure von 0,2 Gew.-%; Wassergehalt 0,1 Gew.-%.

Beispiel 7

152,2 g 4-Hydroxybenzoesäuremethylester wurden suspendiert in 1 385 ml Wasser und protionsweise mit 44,3 g festem Lithiumhydroxidhydrat mit einem LiOH-Gehalt von 54 % unter Kühlung auf 18-20 °C derart versetzt, daß nach Zugabe von 42 g Lithiumhydroxid-hydrat die Zugabe bis zum Vorliegen einer vollständigen Lösung unterbrochen wurde. Aus der Lösung, die einen pH-Wert von 12,0 aufwies, wurde durch Zerstäubungstrocknung rein weißes Lithiumsalz des 4-Hydroxybenzoesäuremethylesters mit einem Gehalt and 4-Hydroxybenzoesäure von 0,4 Gew.-% und einem Gehalt an Wasser von 4,6 Gew.-% gewonnen.

Beispiel 8

250 g 4-Hydroxybenzoesäure-(2-äthylhexyl)-ester wurden in 577 ml Wasser bei 20 °C mit 77,2 g 49,2 %iger Natronlauge in 35 Minuten versetzt und nach Vorliegen einer klaren etwas viskosen Lösung mit 4,1 g 49,2 %iger Natronlauge auf pH 12,6 gebracht. Das gelbliche Produkt der Zerstäubungstrocknung enthält 0,3 Gew.-% 4-Hydroxybenzoesäure und 1,6 Gew.-% Wasser.

Beispiel 9

114 g 4-Hydroxybenzoesäurebenzylester wurden in 263 ml Wasser suspendiert, mit 38,6 g 49,2 %iger Natronlauge unter Einhalten einer Temperatur von 20 °C in 20 Minuten in Lösung gebracht und mit 2,0 g 49,2 %iger Natronlauge auf pH 12,5 gestellt. Das farblose Natriumsalz des 4-Hydroxybenzoesäurebenzylesters, das durch Zerstäubungstrocknung erhalten wurde, enthält 0,3 Gew.-% 4-Hydroxybenzoesäure und 1,3 Gew.-% Wasser.

Beispiel 10

996 g 4-Hydroxybenzoesäureethylester wurden in 1 748 ml Wasser suspendiert und bei 18-20 °C innerhalb von 30 Minuten mit 300 ml 50 %iger Natronlauge bis zu einem Neutralisationsgrad von 0,95 versetzt. In die nach 20 Minuten entstandene Lösung wurden 12 ml Natronlauge (4 %) unter Rühren hinzugefügt. Die Lösung wurde in einem Vakuum-Zweiwalzen-Trockner bei einem Druck von 17 mbar und einer Heizmitteltemperatur von 140 °C zur Trockene gebracht. Das in Schuppen anfallende Natriumsalz des 4-Hydroxybenzoesäureethylesters weist einen Gehalt an 4-Hydroxybenzoesäure von 0,4 Gew.-% und einen Gehalt an Wasser von 0,1 Gew.-% auf.

Beispiel 11

15,2 kg 4-Hydroxybenzoesäuremethylester wurden in 34,8 l Wasser suspendiert und bei 10 bis 20 °C in 30 Minuten mit 5 l 50 %iger Natronlauge (95 % der berechneten Menge) versetzt. Die restlichen 0,23 l 50 %iger Natronlauge wurden in die entstandene Lösung unter Rühren bis zu einem pH-Wert von 11,8 gegeben. Die so bereitete Lösung wurde auf einem Zweiwalzen-Trockner unter Normaldruck bei einer Heizmitteltemperatur von 140 °C getrocknet. Das Endprodukt weist einen Gehalt von 1,0 Gew.-% 4-Hydroxybenzoesäure und 4,7 Gew.-% Wasser auf.

Beispiel 12

Die wie im Beispiel 11 bereitete Lösung des Natriumsalzes des 4-Hydroxybenzoesäuremethylesters wurde in einem Dickschichtkontakttrockner (Schaufeltrockner) bei einem Druck von 40-20 mbar und einer Heizmitteltemperatur von 70-90 °C eingedampft. Das Produkt enthielt 0,2 Gew.-% 4-Hydroxybenzoesäure und 0,03 Gew.-% Wasser.

**Ansprüche**

1. Verfahren zur Herstellung weitgehend wasserfreier und Hydroxybenzoesäure-freier Alkalisalze von Hydroxybenzoesäureestern der Formel

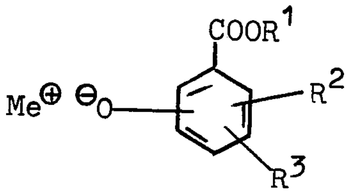

in der

$R^1$ Alkyl, Alkenyl, Cycloalkyl oder Aralkyl bedeutet,

$R^2$ und $R^3$ gleich oder verschieden sind und für Wasserstoff, Halogen Hydroxy, Amino, Alkylamino, Alkyl, Alkoxy, Aralkyl oder Aryl stehen und

Me ein Alkalimetall bedeutet,

dadurch gekennzeichnet, daß man eine Lösung oder Suspension eines Hydroxybenzoesäureesters der Formel

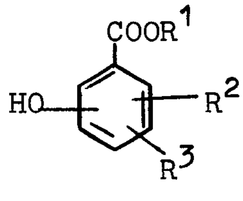

in der

$R^1$, $R^2$ und $R^3$ die oben genannte Bedeutung haben, mit einem Alkalihydroxid bei − 10 bis + 50 °C bis zu einem Neutralisationsgrad von 0,95 bis 1,05 neutralisiert, wobei der Neutralisationsgrad 1,00 den Äquivalenzpunkt der Neutralisation der phenolischen OH-Gruppe durch das Alkalihydroxid bedeutet, und die dabei erhaltene Lösung oder Suspension des Alkalisalzes des Hydroxybenzoesäureesters so rechtzeitig einer an sich bekannten produktschonenden Trocknung zuführt, daß der Gehalt an Hydroxybenzoesäure, bzw. deren Alkalisalz in dieser Lösung oder Suspension den Wert von 1 Gew.-%, bezogen auf die in dieser Lösung oder Suspension enthaltene Menge an Alkalisalz des Hydroxybenzoesäureesters, nicht erreicht.

2. Verfahren nach Anspruch 1, dadurch gekennzeichnet, daß als Lösungs- oder Suspensionsmittel Wasser verwendet wird.

3. Verfahren nach Anspruch 1 und 2, dadurch gekennzeichnet, daß man die Konzentration der Lösung oder Suspension des Alkalisalzes des Hydroxybenzoesäureesters, die der Trocknung zugeführt wird, auf 10 bis 80 Gew.-% einstellt.

4. Verfahren nach Anspruch 1 bis 3, dadurch gekennzeichnet, daß die Konzentration der Lösung des Alkalisalzes des Hydroxybenzoesäureesters die der Trocknung zugeführt wird, auf 0,5 bis 10 Gew.-% unter der Sättigungskonzentration eingestellt wird.

5. Verfahren nach Anspruch 1 bis 3, dadurch gekennzeichnet, daß man die Lösung oder Suspension des Hydroxybenzoesäureesters in einer ersten Stufe bis zu einem Neutralisationsgrad von 0,90 bis 0,97 mit Alkalihydroxid behandelt und in einer zweiten Stufe vollständig neutralisiert.

**Claims**

1. Process for the preparation of alkali metal salts of hydroxybenzoates, which are substantially anhydrous and free from hydroxybenzoic acid and have the formula

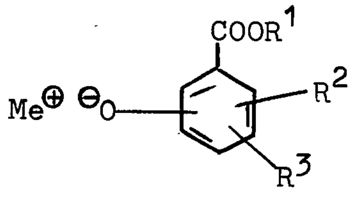

in which

$R^1$ denotes alkyl, alkenyl, cycloalkyl or aralkyl,

$R^2$ and $R^3$ are identical or different and represent hydrogen, halogen hydroxyl, amino, alkylamino, alkyl, alkoxy, aralkyl or aryl and

Me denotes an alkali metal,

characterised in that a solution or suspension of a hydroxybenzoate of the formula

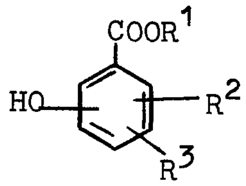

in which

$R^1$, $R^2$ and $R^3$ have the abovementioned meaning, is neutralised with an alkali metal hydroxide at − 10 to + 50 °C until the degree of neutralisation is 0.95 to 1.05, a degree of neutralisation of 1.00 denoting the end point of the neutralisation of the phenolic OH group by the alkali metal hydroxide, and the resulting solution or suspension of the alkali metal salt of the hydroxybenzoate is passed to a drying operation which does not damage the product and is in itself known, after a time such that the content of hydroxybenzoic acid or the alkali metal salt thereof in this solution or suspension does not reach the value of 1 % by weight, relative to the amount of alkali metal salt of hydroxybenzoate contained in this solution or suspension.

2. Process according to Claim 1, characterised in that water is used as the solvent or suspending agent.

3. Process according to Claims 1 and 2, characterised in that the concentration of the solution or suspension of the alkali metal salt of the hydroxybenzoate which is passed to the drying operation is adjusted to 10 to 80 % by weight.

4. Process according to Claims 1 to 3, characterised in that the concentration of the solution of the alkali metal salt of the hydroxybenzoate which is passed to the drying operation is adjusted to 0.5 to 10 % by weight below the saturation concentration.

5. Process according to Claims 1 to 3, characterised in that, in a first stage, the solution or suspension of the hydroxybenzoate is treated with alkali metal hydroxide up to a degree of neutralisation of 0.90 to 0.97 and the neutralisation is brought to completion in a second stage.

**Revendications**

1. Procédé de production de sels alcalins d'esters d'acide hydroxybenzoïque hautement anhydres et dépourvus d'acide hydroxybenzoïque, de formule :

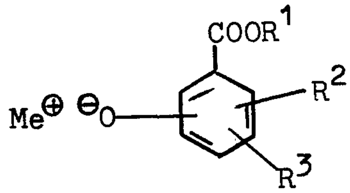

dans laquelle :

$R^1$ est un groupe alkyle, alcényle, cycloalkyle ou aralkyle,

$R^2$ et $R^3$ sont égaux ou différents et représentent de l'hydrogène, un halogène, un groupe hydroxy, amino, alkylamino, alkyle, alkoxy, aralkyle ou aryle, et

Me est un métal alcalin,

caractérisé en ce qu'on neutralise une solution ou suspension d'un ester d'acide hydroxybenzoïque de formule :

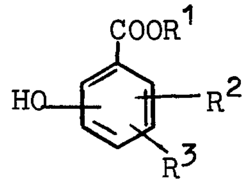

dans laquelle :

$R^1$, $R^2$ et $R^3$ ont la définition mentionnée ci-dessus, avec un hydroxyde alcalin entre − 10 et + 50 °C jusqu'à un degré de neutralisation de 0,95 à 1,05, le degré de neutralisation 1,00 signifiant le point d'équivalence de la neutralisation du groupe OH phénolique par l'hydroxyde alcalin, et on soumet la solution ou suspension ainsi obtenue du sel alcalin de l'ester d'acide hydroxybenzoïque à un séchage connu ménageant le produit, à un moment choisi de manière que la teneur en acide hydroxybenzoïque ou en son sel alcalin dans cette solution ou suspension n'atteigne pas la valeur de 1 % en poids, par rapport à la quantité de sel alcalin d'ester d'acide hydroxybenzoïque contenue dans cette solution ou suspension.

2. Procédé suivant la revendication 1, caractérisé en ce qu'on utilise l'eau comme solvant ou milieu de suspension.

3. Procédé suivant l'une des revendications 1 et 2, caractérisé en ce qu'on ajuste la concentration de la solution ou de la suspension du sel alcalin de l'ester d'acide hydroxybenzoïque que l'on soumet au séchage à une valeur de 10 à 80 % en poids.

4. Procédé suivant les revendications 1 à 3, caractérisé en ce que la concentration de la solution du sel alcalin de l'ester d'acide hydroxybenzoïque que l'on soumet au séchage est ajustée à une valeur de 0,5 à 10 % en poids au-dessous de la concentration de saturation.

5. Procédé suivant les revendications 1 à 3, caractérisé en ce qu'on traite la solution ou suspension de l'ester d'acide hydroxybenzoïque dans une première étape jusqu'à un degré de neutralisation de 0,90 à 0,97 avec un hydroxyde alcalin et on la neutralise totalement dans une seconde étape.